(19) Europäisches Patentamt
European Patent Office
Office européen des brevets

(11) **EP 2 482 713 B1**

(12) **EUROPEAN PATENT SPECIFICATION**

(45) Date of publication and mention
of the grant of the patent:
**31.12.2014 Bulletin 2015/01**

(21) Application number: **10771535.1**

(22) Date of filing: **29.09.2010**

(51) Int Cl.:
*A61B 5/00* (2006.01)   *A61B 8/00* (2006.01)
*A61B 8/08* (2006.01)   *G01N 21/17* (2006.01)
*G01S 15/02* (2006.01)   *G01S 15/89* (2006.01)
*G10K 11/00* (2006.01)

(86) International application number:
**PCT/JP2010/005840**

(87) International publication number:
**WO 2011/040003 (07.04.2011 Gazette 2011/14)**

(54) **PHOTOACOUSTIC MEASURING APPARATUS**

FOTOAKUSTISCHE MESSVORRICHTUNG

APPAREIL DE MESURE PHOTOACOUSTIQUE

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **01.10.2009 JP 2009229653**

(43) Date of publication of application:
**08.08.2012 Bulletin 2012/32**

(73) Proprietor: **Canon Kabushiki Kaisha
Tokyo 146-8501 (JP)**

(72) Inventors:
• **FUKUTANI, Kazuhiko
Tokyo 146-8501 (JP)**
• **KOBAYASHI, Shuichi
Tokyo 146-8501 (JP)**

(74) Representative: **TBK
Bavariaring 4-6
80336 München (DE)**

(56) References cited:
**EP-A1- 2 103 257       WO-A1-2010/074103
WO-A1-2010/074104    US-A1- 2002 173 722
US-A1- 2010 094 561**

• BOOI R C ET AL: "Evaluation of thin compression
paddles for mammographically compatible
ultrasound", ULTRASONICS SYMPOSIUM, 2004
IEEE MONTREAL, CANADA 23-27 AUG. 2004,
PISCATAWAY, NJ, USA,IEEE, vol. 3, 23 August
2004 (2004-08-23), pages 2129-2132,
XP010784418, DOI: DOI:10.1109/ULTSYM.
2004.1418258 ISBN: 978-0-7803-8412-5

## Description

The present invention relates to measuring apparatuses according to the current claims. More particularly, the present invention relates to a measuring apparatus that receives an acoustic wave that has been propagated through a subject and generates image data.

In the medical field, researches on measuring apparatuses that generate image data on the basis of information about the inside of a subject, such as a living body, using light emitted from a light source, such as laser, to irradiate the subject have been actively conducted. A photoacoustic tomography (PAT) apparatus is an example of such a measuring apparatus. In photoacoustic tomography, a subject is irradiated with light emitted from a light source, so that the light is propagated and diffused in the subject. A biological tissue in the subject absorbs energy of the light and generates an acoustic wave (typically an ultrasonic wave). The acoustic wave is received by a probe, so that an electric signal is obtained. The electric signal is mathematically analyzed, and image data is generated on the basis of information regarding an optical characteristic value of the inside of the subject. To efficiently receive the acoustic wave, it is necessary to bring the probe into physical contact with the subject. For this purpose, the probe can be brought into direct contact with the subject using liquid gel or the like that increases adhesion. However, in the case where the subject has a complex external shape, for example, in the case where the subject is a small animal or a human breast, it is difficult to bring a reception surface of the probe into complete contact with a surface of the subject. In such a case, a shape maintaining member, such as a flat plate, is used to, for example, flatten the shape of the subject and the probe is opposed to the subject with the shape maintaining member disposed therebetween. However, since the shape maintaining member has an average sound velocity that differs from that of the subject, in the case where the shape maintaining member is used, the acoustic wave that has been propagated through the subject is refracted at an interface between the subject and the shape maintaining member in accordance with the Snell's law. Therefore, when an ordinary image reconstruction process is performed in which the sound velocity is assumed to be constant, resolution of the image is reduced.

A method for eliminating the influence of the refraction at the interface is described in PTL 1. PTL 1 describes a multifunction machine in which X-ray mammography is combined with an ultrasonic diagnostic apparatus (apparatus that transmits an ultrasonic wave and receives an ultrasonic wave that is reflected in a subject and returned). In X-ray mammography, the subject is pressed by a pressing plate, which functions as the shape maintaining member. An X-ray is caused to pass through the subject, and image data is generated on the basis of information of the subject. In the combination of the X-ray mammography and the ultrasonic apparatus, the ultrasonic wave is received by a probe through the pressing plate. Therefore, to compensate for the refraction of the ultrasonic wave due to the difference in sound velocity between the pressing plate and the subject, a delay process is performed by calculating delay times at a plurality elements included in the probe. Then, signals from the elements are added together.

### Citation List

### Patent Literature

PTL 1: U.S. Patent No. 6,607,489

Prior art which is related to this field of technology can be found in e.g. post-published document WO 2010/074103 A1 disclosing a biological information acquisition apparatus, in post-published document WO 2010/074104 A1 disclosing a biological information acquisition apparatus and biological information acquisition method, and in document US 2010/0094561 A1 disclosing an apparatus and method for processing biological information.

However, it has been found, as a result of diligent studies conducted by the inventors of the present invention, that extremely long time is required to perform the calculation for compensating for the refraction according to the method of PTL 1. According to PTL 1, parameters shown in Fig. 7 satisfies Equation (101) given below in accordance with the Snell's law.

[Math.1]

$$\frac{h_n{}^2}{h_n{}^2 + (f_n - 1)^2} = c_n{}^2 \cdot \frac{(x_n - h_n)^2}{1 + (x_n - h_n)^2} \qquad (101)$$

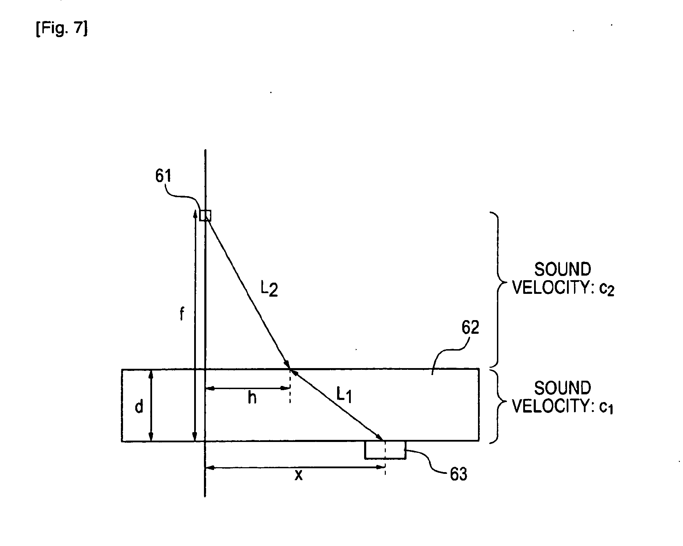

Referring to Fig. 7, reference numeral 61 denotes a voxel of interest (or an electronic focal point), 62 denotes a pressing plate, and 63 denotes a probe. In addition, d is a thickness of the pressing plate, x is a distance between the probe and the voxel of interest along an in-plane direction, f is a distance between the voxel of interest and the probe in a thickness direction of the pressing plate, $c_2$ is an average sound velocity in the subject, $c_1$ is an average sound velocity in the pressing plate, h is a distance between the voxel of interest and a refraction point along the in-plane

direction, $L_2$ is a distance between the voxel of interest and the refraction point, and $L_1$ is a distance between the refraction point and the probe. In Equation (101), for simplicity, the parameters are made dimensionless as follows: $h_n = h/d$, $c_n = c_2/c_1$, $f_n = f/d$, and $x_n = x/d$. $L_2$ and $L_1$ can be determined by calculating $h_n$ from the above equation and then calculating h from $h_n$. In general, in a measuring apparatus that uses an acoustic wave (typically an ultrasonic wave), the time at which the acoustic wave reaches elements differs for each element. Therefore, a delay and sum process is performed in which signals from the elements are added after the delay time is adjusted for each element. Then, image data for each voxel is obtained. The delay time based on the refraction

can be calculated for each voxel from the sound velocities and the distances $L_1$ and $L_2$.

However, a quartic equation must be solved to calculate $h_n$ from Equation (101).

Therefore, a calculation time required for an image reconstruction process including the above-described calculation is at least several times as long as that required for an ordinary image reconstruction process.

Accordingly, the present invention provides a measuring apparatus capable of generating image data in which reduction in resolution due to refraction of an acoustic wave is reduced without performing a complex calculation.

[0011]    - A measuring apparatus according to the invention is defined in the independent claims.

Thus, image data in which reduction in resolution due to refraction of the acoustic wave is small can be obtained without performing a complex calculation for eliminating the influence of the refraction.

## Brief Description of Drawings

[fig.1A]Fig. 1A is a schematic diagram illustrating a measuring apparatus according to a first embodiment of the present invention.

[fig.1B]Fig. 1B is another schematic diagram illustrating the measuring apparatus according to the first embodiment of the present invention.

[fig.2A]Fig. 2A is a schematic diagram illustrating an example of a detected signal at a probe surface.

[fig.2B]Fig. 2B is a schematic diagram illustrating an example of a detected signal at a virtual detection region.

[fig.3A]Fig. 3A is a schematic diagram illustrating an example of a probe capable of forming a virtual detection point.

[fig.3B]Fig. 3B is a schematic diagram illustrating another example of a probe capable of forming a virtual detection point.

[fig.3C]Fig. 3C is a schematic diagram illustrating another example of a probe capable of forming a virtual detection point.

[fig.4]Fig. 4 is a schematic diagram illustrating a measuring apparatus according to a second embodiment of the present invention.

[fig.5]Fig. 5 is a schematic diagram illustrating a measuring apparatus according to a comparative example.

[fig.6A]Fig. 6A illustrates an MIP image of an acoustic wave source in a subject. [fig.6B]Fig. 6B illustrates an MIP image obtained by a method of Comparative Example 1.

[fig.6C]Fig. 6C illustrates an MIP image obtained by a method of Comparative Example 2.

[fig.6D]Fig. 6D illustrates an MIP image obtained by a method of Example 1.

[fig.7]Fig. 7 is a schematic diagram illustrating refraction of an ultrasonic wave caused by a pressing plate according to a related art.

## Description of Embodiments

Measuring apparatuses according to embodiments of the present invention will now be described with reference to the drawings. In the measuring apparatuses according to the embodiments of the present invention, a virtual detection region is set near a subject-side surface of a shape maintaining member. A signal process is performed on the assumption that an acoustic wave is received at the virtual detection region. The virtual detection region will be described in detail below in each embodiment.

Examples of the acoustic wave include waves called a sound wave, an ultrasonic wave, and a photoacoustic wave. For example, the acoustic wave may be an acoustic wave that is generated in a subject when the inside of the subject is irradiated with light (electromagnetic wave), such as a near-infrared ray, or an acoustic wave that is reflected in the subject when an acoustic wave is transmitted toward the inside of the subject. In other words, the measuring apparatus according to an embodiment of the present invention may be a PAT apparatus which irradiates the subject with light and generates image data by receiving a photoacoustic wave generated in the subject with a probe. Alternatively, the measuring apparatus according to an embodiment of the present invention may be an ultrasonic apparatus which generates image data by transmitting an ultrasonic wave toward the inside of the subject and receiving an ultrasonic wave that returns from the inside of the subject.

## First Embodiment

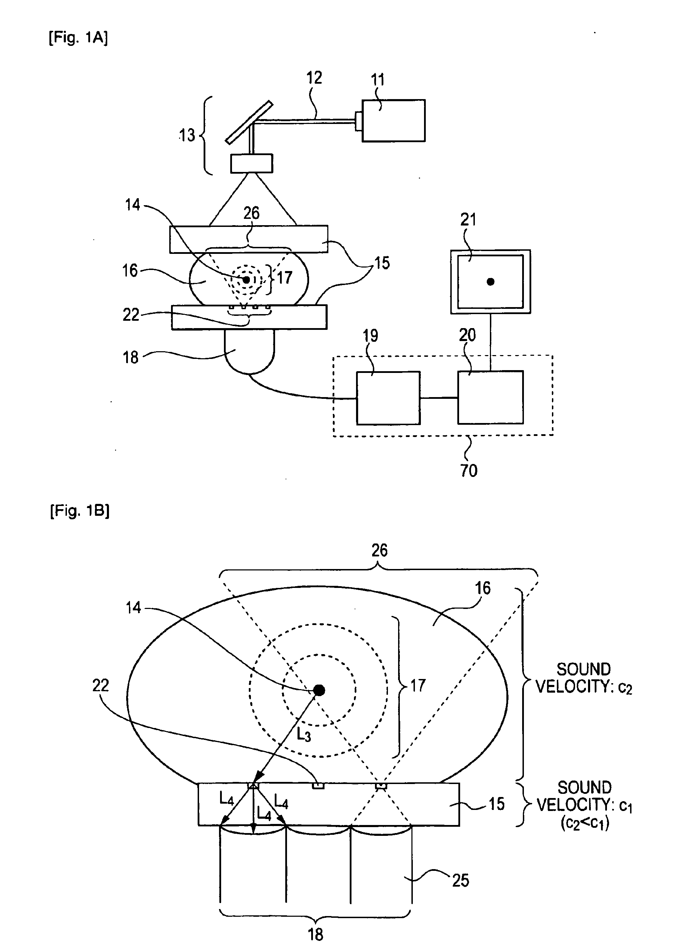

A first embodiment of the present invention will be described with reference to Figs. 1A and 1B. Fig. 1A is a schematic diagram illustrating an example of a measuring apparatus to which the present invention can be applied. Fig. 1B is an enlarged schematic diagram illustrating the structure of a probe and a shape maintaining member for explaining a virtual detection region. In the present embodiment, a PAT apparatus that uses photoacoustic tomography is described as an example. The PAT apparatus generates image data by receiving an acoustic wave generated in a subject when the subject is irradiated with a light pulse.

The PAT apparatus according to the present embodiment includes a light source 11, an optical component 13, a pressing plate 15 that serves as a shape maintaining member, a probe 18, and a processing unit 70. Image data is generated by the processing unit 70, and is displayed by a display device 21. The processing unit 70 includes a signal processor 19 and an image reconstruction processor 20. Light 12 is emitted from the light source 11, guided by the optical component 13, such as a mirror and a lens, and is incident on a subject 16, such as a living body. A part of energy of the light 12 that propagates through the subject 16 is absorbed by a light absorber 14 (which finally serves as an acoustic wave source), such as a blood vessel. Accordingly, an acoustic wave 17 (typically an ultrasonic wave) is generated as a result of thermal expansion of the light absorber 14. The generated acoustic wave 17 is received by the probe 18, and is subjected to a process for generating image data (image reconstruction process).

The light source 11 emits light with a particular wavelength that can be absorbed by a particular component of the living body. The light source can be provided integrally with the measuring apparatus according to an embodiment of the present invention, or be provided separately from the measuring apparatus. The light source includes at least one pulsed light source capable of emitting pulsed light with the order of several nanoseconds to several hundreds of nanoseconds. A laser that is capable of providing a large output can be used as the light source. However, a light emitting diode or the like can be used in place of the laser. Various lasers such as a solid laser, a gas laser, a dye laser, and a semiconductor laser may be used as the laser. Emission timing, waveform, strength, etc., of the light are controlled by the signal processor 19 or a control unit (not shown).

The optical component 13 includes, for example, a mirror that reflects the light and a lens that collects the light, enlarges the light, or changes the shape of the light. In place of the mirror and lens, an optical waveguide, for example, may be used as the optical component. The optical component is not particularly limited as long as the light 12 emitted from the light source can be formed into a desired shape and be incident on the subject 16. In general, the light can be diffused by a lens so that the area thereof is somewhat increased. In addition, the region in which the subject is irradiated with the light can be movable along the subject. In other words, the measuring apparatus according to an embodiment of the present invention can be structured such that the light emitted from the light source is movable along the subject. In the case where the light is movable, a large area can be irradiated with the light. In addition, the region in which the subject is irradiated with the light (light with which the subject is irradiated) can be moved in synchronization with the probe 18. A method for moving the region in which the subject is irradiated with the light may be a method using a movable mirror or the like or a method in which the light source itself is mechanically moved.

The measuring apparatus according to an embodiment of the present invention can be used to diagnose a malignant tumor, a blood vessel disease, etc., of a human or an animal. Therefore, the subject 16 may be a diagnosis target, such as a breast, finger, limb, etc., of a human or animal body. In photoacoustic tomography, the light absorber (or acoustic wave source) 14 is a component of the subject that has a high absorption coefficient. For example, in the case where the target of measurement is a human body, oxygenated or reduced hemoglobin, a blood vessel including a large amount of oxygenated or reduced hemoglobin, a malignant tumor including a large number of new blood vessels, etc., correspond to the light absorber (or acoustic wave source) 14. In the case where the measuring apparatus is used as an ultrasonic apparatus, the acoustic wave source 14 is a component of the subject that has an acoustic impedance that differs from those of the surrounding components.

The shape maintaining member 15 is provided between the subject and the probe. The shape maintaining member 15 is used to maintain the shape of at least a part of the subject 16, and may have a function of retaining or pressing the subject 16. The shape maintaining member 15 may be a flat plate, a pressing plate, or a parallel plate. To increase the efficiency in receiving the acoustic wave, the shape maintaining member 15 can be made of a material having an acoustic impedance that is close to that of the subject. In the case where the subject is a breast or the like, a shape maintaining member made of polymethylpentene can be used. The shape maintaining member can be plate-shaped. However, the shape maintaining member can be formed in any shape as long as a reception surface of the probe and a surface of the shape maintaining member can be brought into tight contact with each other. In the case where the shape maintaining member is plate shaped, the shape maintaining member can be as thin as possible in consideration of the attenuation of the acoustic wave, but must be thick enough to prevent deformation thereof. Typically, the thickness of the shape maintaining member is about 5 mm to 10 mm. To eliminate an air space between the shape maintaining member and the subject or between the shape maintaining member and the probe, gaps therebetween can be filled with liquid (not shown) such as acoustic gel having an acoustic impedance close to that of the shape maintaining member (about 1.35 * $10^6$ $Kg/m^2s$ when, for example, the subject is a breast) or water.

The probe 18 includes one or more elements, each of which receives the acoustic wave and converts the acoustic wave into an electric signal. More specifically, the probe 18 includes transducers that use the piezoelectric phenomenon, transducers that use the optical resonance, or transducers that use the capacitance change. The probe 18 is not limited as long as the acoustic wave can be received and converted into an electric signal. A probe including a plurality of elements 25 that receive the acoustic wave is illustrated as an example in Figs. 1A and 1B. However, a probe in which a plurality of elements are two-dimensionally arranged can be used. In the case where the elements are two-dimensionally arranged, the acoustic wave can be received at a plurality of locations at the same time. As a result, the receiving time can be reduced and the influence of vibration or the like of the subject can be reduced. Signals similar to those obtained by a plurality of elements that are two-dimensionally or one-dimensionally arranged may also be obtained by operating a single element.

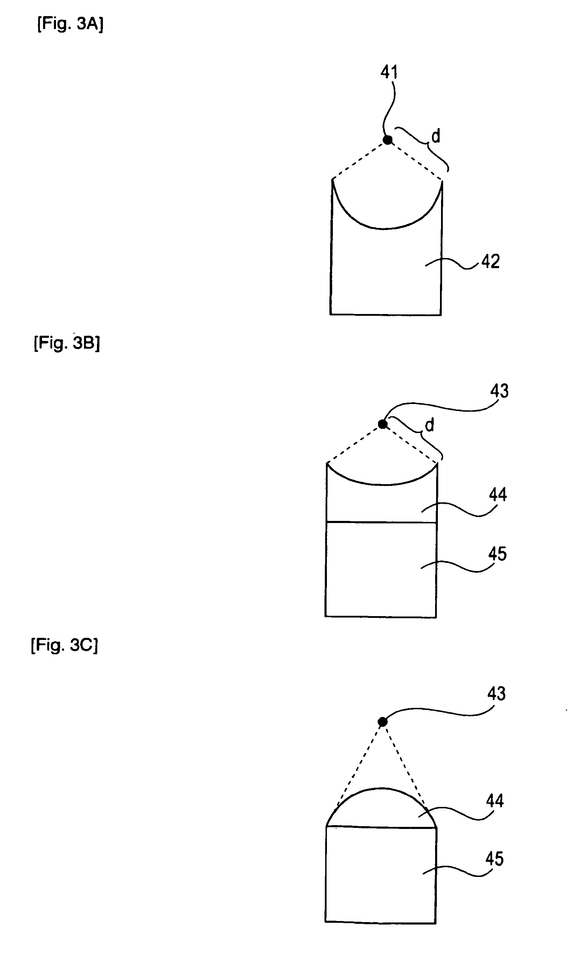

For each of the elements included in the probe 18, a virtual detection region is set near the subject-side surface of the shape maintaining member 15. In the present embodiment, the probe may include an element having a circular concave reception surface, as illustrated in Fig. 3A. Alternatively, the probe may include an element having a concave acoustic lens attached to a front reception surface of the element, as illustrated in Fig. 3B or an element having a convex acoustic lens attached to a front reception surface of the element, as illustrated in Fig. 3C. The convex lens is used if the sound velocity of the lens material is smaller than that of the shape maintaining member, and the concave lens is used if the sound velocity of the lens material is larger than that of the shape maintaining member. For example, in the case where an acoustic lens is made of a silicon rubber having a sound velocity of 1,000 m/s and a sound velocity in an area surrounding the acoustic lens is 1,500 m/s, the acoustic lens is formed in a convex shape.

In the present embodiment, the "virtual detection region" refers to a region which is near the subject-side surface of the shape maintaining member 15 and at which a distance from the reception surface is acoustically constant. The region at which the distance from the reception surface is acoustically constant is a region that satisfies the condition that when an acoustic wave is emitted from the region, the acoustic wave reaches the reception surface at the same time irrespective of a position on the reception surface at which the acoustic wave arrives. Theoretically, the position at which the distance from all of the positions on the reception surface is constant is at a single point. For example, in the case where an element having a concave reception surface is used as illustrated in Fig. 3A, the position at which the distance from the reception surface is acoustically constant is at the center of curvature of the reception surface. However, the element has a finite width in practice and it is difficult to determine the position on the reception surface at which the acoustic wave is received. Therefore, the "virtual detection region" may have a width. Thus, the "virtual detection region" is defined as a region centered on a single ideal point at which the distance from the reception surface is acoustically constant (that is, a region at which it can be assumed that the distance from the reception surface is acoustically constant). This region is a region (focus zone) in which an acoustic wave is collected if the acoustic wave is transmitted from the reception surface of the element.

The virtual detection region will now be described in more detail. In the present embodiment, virtual detection regions 22 will be described with reference to Figs. 1A and 1B. As described above, each virtual detection region 22 is a region at which it can be assumed that the distance from the corresponding reception surface is constant, and does not exist in practice. In addition, from the viewpoint of resolution, each virtual detection region can be small relative to the area of the reception surface of each element 25 of the probe. Therefore, in the present embodiment, the region at which it can be assumed that the distance from the reception surface is acoustically constant (that is, the virtual detection region) is defined as a region at which sensitivity is higher than or equal to one-half of the sensitivity (maximum sensitivity) at the ideal point (center of the virtual detection region) at which the distance from the reception surface is acoustically constant. The sensitivity is the level of the received acoustic wave, that is, the level of an output signal relative to the level of an input signal. The size of cross section of the region (focus zone) in which an acoustic wave is collected if the acoustic wave is transmitted from the reception surface of each element can be approximated by the first-kind first-order Bessel function. Therefore, assuming that there is a circular virtual detection region, a diameter L of the virtual detection region can be calculated by Equation (102) given below from a frequency f of the acoustic wave to be received, a sound velocity $c_1$ of the shape maintaining member, a distance d between the reception surface of the corresponding element and the point at which the distance from the reception surface is acoustically constant, and a diameter D of the reception surface of the element (when it is assumed that the reception surface is circular).

[Math.2]

$$L = 1.02 \cdot d \cdot c_1 / (f \cdot D) \qquad (102)$$

In the case of flat-type measurement illustrated in Figs. 1A and 1B, the resolution can be increased compared to that in the related art if the diameter L of each virtual detection region is smaller than the diameter D of the reception surface of each element. For example, assuming that a 10-mm-thick flat plate made of polymethylpentene (sound velocity: 2,200 m/s) is used as the shape maintaining member, when the frequency of the acoustic wave is 5 MHz and the diameter of the reception surface of each circular element is 5 mm, the diameter of each virtual detection region can be calculated as about 0.9 mm. Thus, the diameter of each virtual detection region is smaller than the diameter of the reception surface of each element. If the area of each virtual detection region is smaller than the area of the reception surface of each element as described above, the area of the reception surface at which the acoustic wave 17 is received can be effectively reduced. Therefore, in the case of flat-type measurement, the resolution can be easily increased. The resolution described herein is the resolution in a direction parallel to the reception surface.

In the embodiments of the present invention, a region near the subject-side surface of the shape maintaining member is a region including a physical interface between the shape maintaining member 15 and the subject 16 and determined by Equation (103) given below. A region that can be set as the virtual detection region is a three-dimensional focus zone. A dimension $F_z$ of this region in a direction perpendicular to the interface can be determined by Equation (103) from the frequency f of the acoustic wave to be received, the sound velocity $c_1$ of the shape maintaining member, a distance $S_F$ between the virtual detection area and the reception surface of the element that is normalized by the sound velocity, and the diameter D of the reception surface of the element (when it is assumed that the reception surface is circular).

[Math.3]

$$F_z = \frac{D^2 \cdot f}{4c_1} \cdot S_F{}^2 \cdot \left[ \frac{2}{1 + 0.5 S_F} \right] \qquad (103)$$

Thus, in the embodiments of the present invention, a region which includes the physical interface between the shape maintaining member and the subject and in which a distance from the interface is in the range of plus or minus $F_z$ is defined as the region near the subject-side surface of the shape maintaining member.

Next, the signal process performed by the processing unit 70 illustrated in Fig. 1A will be described. The processing unit 70 according to the present embodiment includes the signal processor 19 and the image reconstruction processor 20. The signal processor 19 amplifies an electric signal (first electric signal) output from each element and converts the electric signal, which is an analog signal, into a digital signal. The signal processor 19 typically includes an amplifier, an A/D converter, a field programmable gate array (FPGA) chip, and other components. The FPGA is used to control an amplification factor of the amplifier and signal reception timing. The image reconstruction processor 20 subjects the signal that has been converted into the digital signal by the signal processor 19 to a process which will be described below. Then, the image reconstruction processor 20 performs the image reconstruction process to generate the image data.

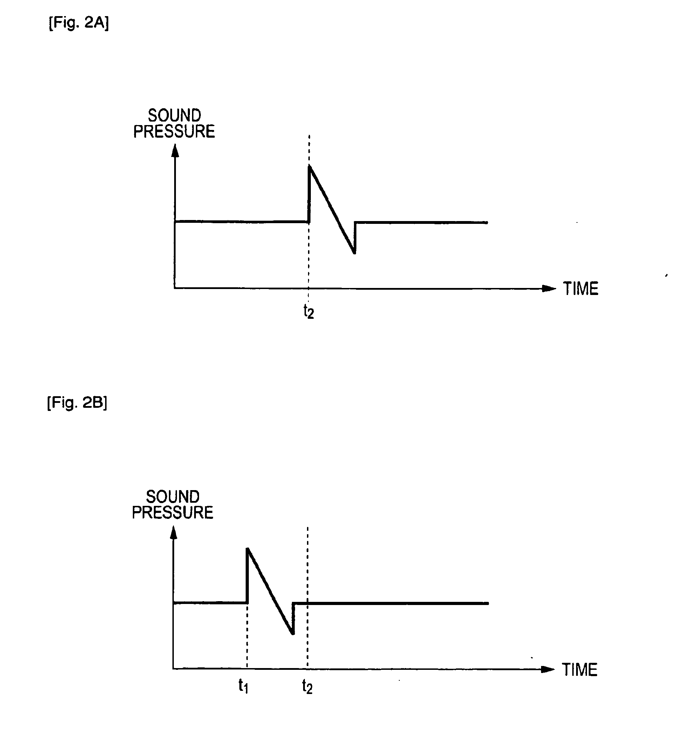

The signal process performed by the image reconstruction processor 20 will now be described. Referring to Figs. 2A and 2B, a process of converting the first electric signal (detected signal) that has been converted into the digital signal by the signal processor 19 into a second electric signal will be described. The second electric signal is assumed to have been obtained at each virtual detection region 22. Fig. 2A illustrates an example of a detected signal (first electric signal) of an acoustic wave output from one of the elements of the probe 18 illustrated in Fig. 1B. The detected signal is a signal that has been converted into a digital signal by the signal processor 19 and transmitted to the image reconstruction processor 20. Fig. 2B illustrates an example of a detected signal (second electric signal) of an acoustic wave based on the assumption that the acoustic wave has been received at the corresponding virtual detection region 22. In Figs. 2A and 2B, the horizontal axis shows the measurement time and the vertical axis shows the value that is proportional to the sound pressure of the detected signal. In general, it is known that a detected signal of an acoustic wave generated by a spherical absorber by which light has been uniformly absorbed is N-shaped, as illustrated in Figs. 2A and 2B, when the acoustic wave is received by an ideal probe.

Referring to Fig. 2A, when the time at which the subject is irradiated with light is 0 seconds, it can be assumed that the time at which the acoustic wave is emitted from the light absorber in the subject is also 0 seconds. In this case, the time $t_2$ at which the first electric signal, which is the N-shaped signal, is output is equal to the time from when the acoustic wave is generated in the subject to when the acoustic wave is received by the element and converted into the electric signal (first electric signal), that is, the time from when the subject is irradiated with light to when the acoustic wave generated in the subject reaches the reception surface of the element (first time). In other words, the time $t_2$ at which the first electric signal is obtained is the sum of the time $t_3$ in which the acoustic wave propagates through the subject and the time $t_4$ (second time) in which the acoustic wave travels from the virtual detection region to the reception surface of the element.

$$t_2 = t_3 + t_4 \ (104)$$

More specifically, $t_2$ is the sum of the time calculated by dividing the distance $L_3$ from the light absorber 14 to the virtual detection region 22 by the average sound velocity $c_2$ of the subject and the time calculated by dividing the distance $L_4$ from the virtual detection region 22 to the reception surface of the element 25 by the average sound velocity $c_1$ of the shape maintaining member 15 in Fig. 1B. Here, the gap at the interface between the shape maintaining member 15 and the element is filled with gel having the same sound velocity as that of the shape maintaining member or by a portion of the shape maintaining member itself. Therefore, the time in which the acoustic wave travels from the virtual detection region to the reception surface of the element can be calculated as the time obtained by dividing $L_4$ by $c_1$.

The time $t_1$ at which the N-shaped signal is output as the detected signal of the acoustic wave to be received at the virtual detection region 22 illustrated in Fig. 2B is equal to the time $t_3$ in which the acoustic wave generated by the light absorber in the subject travels through the subject, that is, the time calculated by dividing the distance $L_3$ from the light absorber 14 to the virtual detection region 22 by the average sound velocity $c_2$ of the subject. That is, the first electric signal illustrated in Fig. 2A can be converted into the second electric signal illustrated in Fig. 2B by subtracting the second time in which the acoustic wave travels from the virtual detection region to the reception surface of the element (time $t_4$ calculated by dividing the distance $L_4$ from the virtual detection region 22 to the element 25 by the average sound velocity $c_1$ of the shape maintaining member) from the first time ($t_2$).

$$t_1 = t_3 = t_2 - t_4 \ (105)$$

In the present embodiment, the acoustic wave emitted from the acoustic wave source also reaches regions in the shape maintaining member other than the virtual detection region in practice. However, the acoustic wave that reaches the regions other than the virtual detection region is at a large angle with respect to the reception surface of the element, and only a small part of the acoustic wave is received by the element. Therefore, when the delay and sum process is performed, the reception sensitivity is significantly reduced. As a result, the reconstructed image data is not largely influenced. Thus, according to the present embodiment, a function as a filter that eliminates the acoustic wave that reaches the regions in the shape maintaining member other than the virtual detection region is provided by forming the reception surface of the element in a concave shape or providing a concave or convex acoustic lens on the reception surface.

In general, the distance $L_4$ between the reception surface of the element and the virtual detection region 22 and the average sound velocity $c_1$ of the shape maintaining member are known in advance. Therefore, as described above, the second electric signal, which is the detected signal of the acoustic wave at the virtual detection region 22, can be calculated from the first electric signal, which is the detected signal of the acoustic wave at the element, simply by determining the first time from the first electric signal and calculating the difference between the first time and the second time. Thus, according to the embodiments of the present invention, unlike the method of PTL 1, the detected signal at the virtual detection region 22 can be obtained without solving a quartic equation. Therefore, the image reconstruction process can be quickly performed.

In addition, the image reconstruction processor 20 included in the measuring apparatus to which the present invention can be applied also subjects the second electric signal generated by the above-described method to a calculation process for generating image data. More specifically, a workstation or the like is typically used to perform the process of converting the first electric signal into the second electric signal and the image reconstruction process using the second electric signal on the basis of pre-programmed software. For example, the software includes two modules, which are a signal processing module for performing the process of converting the first electric signal into the second electric signal and a noise reduction process and an image reconstruction module for performing the image reconstruction process. In the process of converting the first electric signal into the second electric signal, the time ($t_4$) in which the acoustic wave travels from the virtual detection region to the element is provided as an offset for the detected signal, as described above. In the case of photoacoustic tomography, the signal received at each position is subjected to the noise reduction process and other processes as preprocessing before the image reconstruction process. These processes can be performed by the signal processing module. The image reconstruction module performs the image reconstruction process in which back projection in time domain or Fourier domain, which are commonly used as a tomography technique, is performed as an image reconstruction algorithm. The signal processor 19 and the image reconstruction processor 20 can be integrated with each other. In such a case, the image data can be generated by hardware instead of performing software processes using the workstation. The process for converting the signal output from the probe 18 into the detected signal assumed to have been output from the virtual detection region 22 can be performed by the FPGA included in the signal processor 19. In the case where the probe in which a plurality of elements are arranged is used, the virtual detection region is provided for each element. Accordingly, the processing unit 70 generates the image data using a plurality of second electric signals.

The display device 21 displays an image output from the image reconstruction processor 20, and a liquid crystal display or the like is typically used as the display device 21.

By using the measuring apparatus having the above-described structure, even when a shape maintaining member having a sound velocity that largely differs from that of the subject is provided between the subject and the probe, an image having a high resolution can be quickly obtained compared to the method of the related art.

**Second Embodiment**

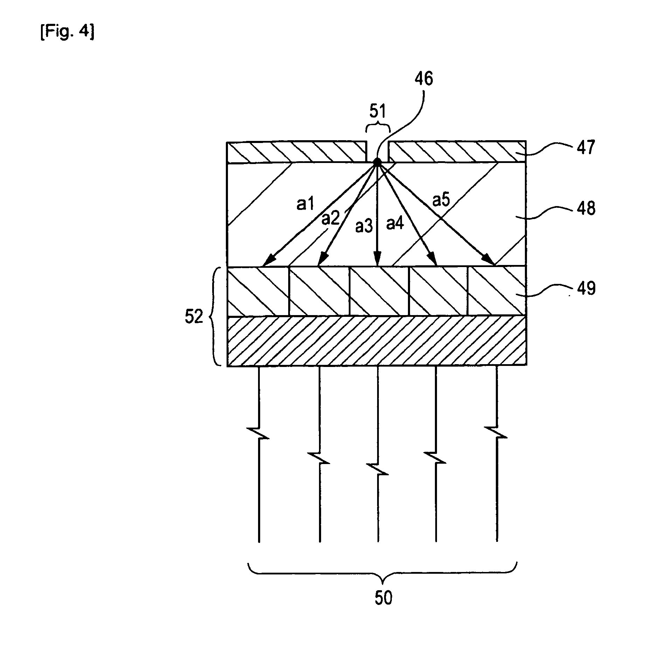

In a second embodiment, as illustrated in Fig. 4, a filter 47 having an opening 51 is provided on a shape maintaining member 48. The second embodiment differs from the first embodiment in that the opening 51 defines a virtual detection region 46 and an element is divided into a plurality of reception surfaces.

As illustrated in Fig. 4, the filter 47 having the physical opening 51 and made of a material that reflects or absorbs ultrasonic reflective waves is provided on the shape maintaining member 48. An acoustic wave that passes through the opening 51 is received by an element 52. The element 52 is divided into a plurality of reception surfaces 49. The acoustic wave is received by the reception surfaces and is converted into a plurality of electric signals (detected signals) 50. Then, a processing unit performs a delay process on the basis of distances a1 to a5 from the opening to the reception surfaces and adds the detected signals 50. The delay process is performed to compensate for differences between arrival times at which the acoustic wave reaches the reception surfaces. In the delay process, one of the reception surfaces is set as a reference reception surface, and the detected signal output from the reference reception surface is used as a reference detected signal. In the present embodiment, the detected signals for which the differences between the arrival times of the detected signals at the respective reception surfaces are compensated for by the delay process are added together to obtain an addition signal. This addition signal is used as a detected signal output by the element (that is, a signal corresponding to the first electric signal in the first embodiment). A first time from when the subject is irradiated with light to when the acoustic wave reaches the reception surfaces of the element (that is, the time at which the first electric signal is output) is the time from when the subject is irradiated with light to when the acoustic wave reaches the reference reception surface and is converted into the detected signal. In addition, the time in which the acoustic wave travels from the virtual detection region to the reception surfaces of the element (second time) is the time in which the acoustic wave travels from the virtual detection region to the reference reception surface of the element. For example, in the case where the delay process is performed using the distance a1 as a reference in Fig. 4, the second time is the time in which the acoustic wave travels along the distance a1 through the shape maintaining member.

The detected signal output by the element (first electric signal) can be converted into the detected signal that is assumed to have been received at the virtual detection region (second electric signal) by using the difference between the first time and the second time, similar to the first embodiment.

As described above, according to the present embodiment, the physical opening 51 is formed at the surface of the shape maintaining member at a position corresponding to the center of the element. Thus, the virtual detection region 46 is defined at a position near the subject-side surface of the shape maintaining member. The acoustic wave that reaches regions other than the opening is reflected or absorbed by the filter, and does not reach the element. Also in the present embodiment, the size of the virtual detection region 46 can be small relative to the reception surface of the element. Therefore, the opening can be formed such that a region in which sensitivity is higher than or equal to one-half of the sensitivity (maximum sensitivity) at the center of the opening serves as the virtual detection region.

As described above, in the present embodiment, a probe having any type of structure can be used as long as the opening that defines the virtual detection region can be formed near the subject-side surface of the shape maintaining member. In the measuring apparatus according to the present embodiment, components other than the probe and the shape maintaining member may have structures similar to those in the first embodiment.

**Third Embodiment**

In the first and second embodiments, the PAT apparatus using photoacoustic tomography that receives an acoustic wave generated in a subject in response to irradiation of the subject with light is described in detail. However, the same principle can also be applied to a measuring apparatus (ultrasonic apparatus) which generates image data by transmitting an acoustic wave (typically an ultrasonic wave) from a probe and receiving an acoustic wave that has been reflected in the subject. In this case, similar to the first and second embodiments, the virtual detection region can be used in a receiving process. In addition, also in a process of transmitting the acoustic wave, the virtual detection region can be used as an virtual transmission region. The above-described virtual detection region directly serves as the virtual transmission region, and the ultrasonic wave can be transmitted as if it is transmitted from the virtual transmission region (virtual detection region). As a result, disturbance in the wavefront due to the refraction caused by the shape maintaining member can be reduced also in the transmitted acoustic wave.

An acoustic-wave transmitter that transmits the acoustic wave is integrated with the probe that receives the acoustic wave. In other words, in the present embodiment, the probe 18 illustrated in Figs. 1A and 1B not only receives an acoustic wave and converts the acoustic wave into an electric signal but also converts an electric signal into an acoustic wave and transmits the acoustic wave. In the present embodiment, the signal output by the element (first electric signal) is converted into the signal that is assumed to have been output at the virtual detection region (second electric signal) as follows. That is, the second electric signal is determined by subtracting the time in which the acoustic wave reflected in the subject travels from the virtual detection region to the element (second time) from one-half of the time from when the acoustic wave is transmitted by the probe to when the reflected acoustic wave is received by the probe (first time). Then, the image data is generated using the second electric signal.

The embodiments of the present invention can also be carried out by the following process. That is, a software (program) that realizes the functions of the above-described embodiments may be supplied to a system or an apparatus via a network or from various storage media, and be read out and executed by a computer (or CPU, MPU, etc.) included in the system or the apparatus.

## Example 1

The results of simulations in which the embodiments of the present invention were carried out will now be described. It is assumed that a probe includes an element having a concave reception surface as illustrated in Fig. 3A. In Example 1, a simulation was carried out under the following calculation conditions. That is, a subject was assumed to be a 4 cm * 4 cm * 4 cm cube at the center of which a columnar acoustic wave source, whose diameter is 0.05 cm at the bottom surface and height is 2 cm, is disposed such that the axial direction of the columnar acoustic wave source is parallel to a shape maintaining member. The average sound velocity of the subject was set to 1,500 m/s. The shape maintaining member was assumed to be a flat-plate-shaped member made of polymethylpentene and have a thickness of 1 cm and a planar size of 4 cm * 4 cm. The average sound velocity of the shape maintaining member was set to 2,200 m/s. The shape maintaining member was assumed to be disposed between the probe and the subject, as illustrated in Fig. 1B. The probe was assumed to be a single-element-type probe which includes a single reception surface (that is, a single element) and which has a diameter of 0.6 cm and a center frequency of 5 MHz. The reception surface was assumed to be a focus-type concave circular surface, and the focus distance was set to 1 cm so that the focal point is at the interface between the shape maintaining member and the subject. In this case, the diameter of the circular virtual detection region can be calculated as 0.075 cm from Equation (102). To obtain an image of the region of 4 cm * 4 cm * 4 cm, it was assumed that the probe was moved at intervals of 2 mm and the acoustic wave was measured at each of 20 points * 20 points in X and Y directions. The sampling frequency of the measurement process was 20 MHz, and the number of measurement points was 1,280. Universal back projection, which is a known time domain method, was used as the image reconstruction method.

The following two calculations were performed as comparative examples.

## COMPARATIVE EXAMPLE 1

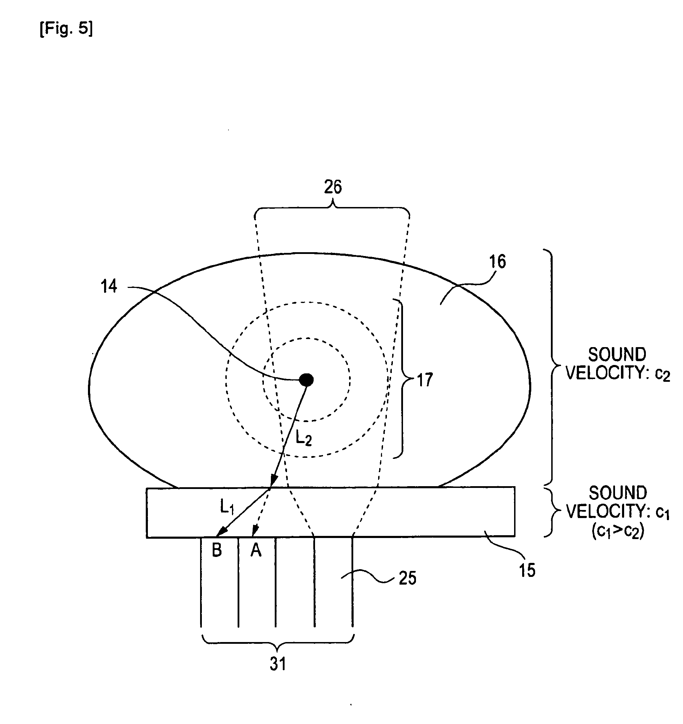

Fig. 5 is a diagram illustrating the effect of refraction of an acoustic wave caused by a plate. Fig. 5 illustrates an acoustic wave source 14, a flat plate 15 that functions as a shape maintaining member, a subject 16, an acoustic wave 17 generated from the source, a probe 31, and an element 25 in the probe. The average sound velocities of the subject 16 and the flat plate 15 are $c_2$ and $c_1$, respectively, and $c_2 < c_1$ is satisfied. The element 25 is an ordinary flat-type element which is not a focus type. The flat-type element is capable of receiving a part of the acoustic wave in a region 26 shown by the dashed lines. In this structure, the acoustic wave 17 generated by the acoustic wave source 14 is refracted at an interface between the subject and the flat plate owing to the difference in sound velocity between the subject and the flat plate. Referring to Fig. 5, in the case where the average sound velocity of the flat plate is equal to that of the subject, no refraction occurs. Therefore, the acoustic wave is received at point A. If there is a difference in sound velocity between the flat plate and the subject, the acoustic wave is received at point B owing to the refraction.

In Comparative Example 1, it was assumed that the reception surface for receiving the acoustic wave was a circular flat plate with a diameter of 2 mm. The signal obtained by the probe was subjected to the image reconstruction process in which the refraction due to the flat plate was ignored and only the sound velocity of the subject was used.

## COMPARATIVE EXAMPLE 2

In Comparative Example 2, it was assumed that the reception surface of the probe for receiving the acoustic wave was a circular flat plate with a diameter of 2 mm, and the image reconstruction process was performed by compensating for the refraction of the acoustic wave due to the flat plate by the method described in PTL 1, which is an example of the related art. More specifically, the distances $L_2$ and L, in Fig. 5 were calculated by Equation (101), and the delay process was performed in consideration of the sound velocities of the flat plate and the subject.

**Simulation Results**

Three images were obtained as results of the image reconstruction process performed in accordance with Comparative Examples 1 and 2 described above and Example 1 in which the virtual detection region was used.

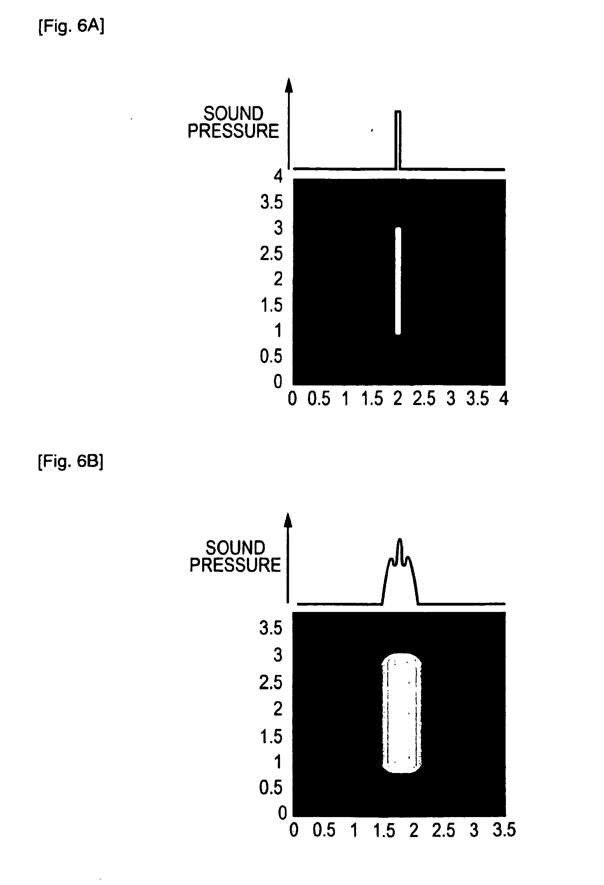

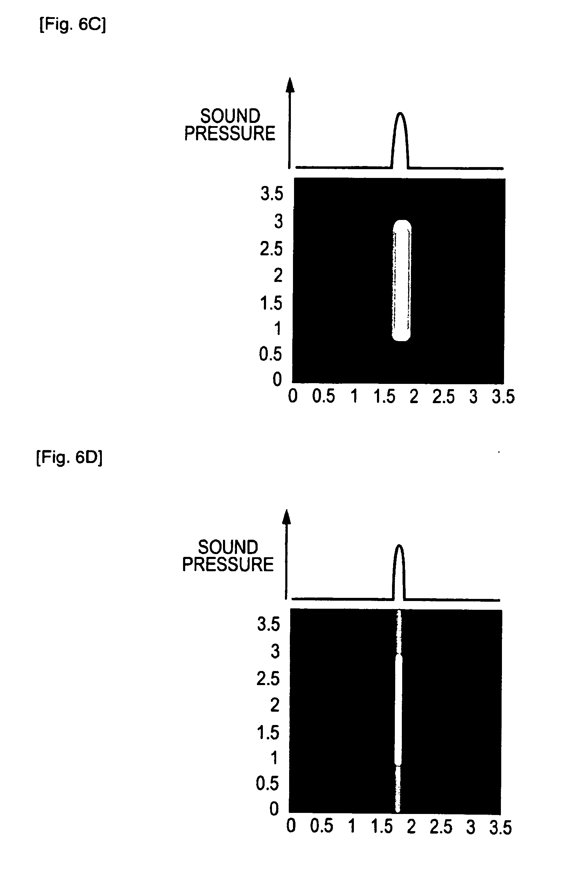

The images obtained by the image reconstruction process will be described with reference to Figs. 6A to 6D. All of the images illustrated in Figs. 6A to 6D are maximum intensity projection (MIP) images in which voxel values with the maximum intensities are projected in the direction of the probe. White regions show the regions with high sound pressures. The graphs illustrated above the images are obtained by plotting the sound pressure at the center of each image. Fig. 6A illustrates an MIP image of the acoustic wave source in the subject. In the simulations, it is determined that an image has a high resolution if the image is close to the image in Fig. 6A. Fig. 6B is an MIP image obtained by the method of Comparative Example 1, Fig. 6C is an MIP image obtained by the method of Comparative Example 2, and Fig. 6D is an MIP image obtained by the method of Example 1. When Fig. 6B is compared with Fig. 6A, the image in Fig. 6B is obviously unclear and therefore the resolution of the image is considerably low. This is due to the refraction of the acoustic wave, as described above. In Fig. 6C, although the width of the reconstructed image of the acoustic wave source is somewhat increased, the resolution is higher than that in Fig. 6B. In contrast, in Fig. 6D, the image is only slightly blurred and the shape of the acoustic wave source is clearly reproduced. As is clear from the comparison between Figs. 6C and 6D, the resolution of the image obtained by the method according to Example 1 is slightly higher than the resolution of the other image. This is because the width of the virtual detection region was 0.75 mm and was smaller than 2 mm, which was the reception width of the element used in Comparative Example 2. Thus, by using the method according to Example 1, an image with a higher resolution than that of an image obtained by the method according to PTL 1 can be obtained using an element having a reception surface with the same size as that in PTL 1.

In addition, regarding the time required for the image reconstruction process, the time required to obtain the image illustrated in Fig. 6C was about 20 times longer than the time required to obtain the image illustrated in Fig. 6C. As described above, an image in which reduction in resolution due to refraction is reduced can be quickly obtained by using the measuring apparatus according to Example 1.

**Example 2**

As Example 2, a result of a simulation in which a probe is assumed to have a convex acoustic lens on a reception surface thereof, as illustrated in Fig. 3C, will now be described. In this example, the simulation was carried out under the following calculation conditions. That is, the subject and the shape maintaining member were assumed to be similar to those in Example 1. The probe was assumed to include a single element and have a diameter of 0.6 cm and a center frequency of 5 MHz. The reception surface of the element was assumed to have a flat circular shape, so that the acoustic wave cannot be focused by the element itself. The convex acoustic lens was assumed to be disposed between a flat plate that functions as a shape maintaining member and the probe. The acoustic lens was assumed to be made of a silicon rubber having a sound velocity of 1,000 m/s and arranged such that the focal point is at the interface between the plate and the subject. In this case, the diameter of the circular virtual detection region can be calculated as 0.075 cm from Equation (102). Similar to Example 1, to obtain an image of the region of 4 cm * 4 cm * 4 cm, it was assumed that the probe was moved at intervals of 2 mm and the acoustic wave was measured at each of 20 points * 20 points in X and Y directions. The sampling frequency of the measurement process was 20 MHz, and the number of measurement points was 1,280. Universal back projection, which is a known time domain method, was used as the image reconstruction method. Then, similar to Example 1, the simulation result was compared with the other results.

The image obtained in Example 2 was substantially similar to that in Example 1. As a result, an image in which reduction in resolution due to the refraction is reduced can be quickly obtained by using the measuring apparatus according to Example 2. Also in the case where a plurality of elements were arranged and the acoustic wave was received by the elements simultaneously without moving the probe, a similar image was obtained.

**Example 3**

Next, an example of a measuring apparatus which is capable of transmitting and receiving an acoustic wave and which uses an ultrasonic diagnostic technique will be explained. The measuring apparatus transmits an acoustic wave (typically an ultrasonic wave), receives an acoustic wave (ultrasonic wave) reflected in the subject, and generates image data on the basis of the reflected acoustic wave.

A simulation was performed to confirm whether or not an virtual transmission region of the acoustic wave can be formed. Also in this simulation, similar to Example 1, a shape maintaining member was assumed to be a flat plate made of polymethylpentene and have a thickness of 1 cm and a size of 4 cm * 4 cm. Here, it was assumed that the probe is capable of both transmitting and receiving an acoustic wave. The probe was assumed to be a single-element-type probe which includes a single reception surface (that is, a single element) and which has a diameter of 0.6 cm and a center frequency of 5 MHz. The reception surface was set to a focus-type concave circular surface, and the focus distance was set to 1 cm so that the focal point is at the interface between the polymethylpentene plate and the subject. The intensity distribution of the acoustic wave transmitted from the above-described probe was calculated. As a result, the transmitted acoustic wave was focused at an end of the polymethylpentene plate, and the width of the focused acoustic wave was about 0.75 mm. A plurality of probes similar to the above-described probe may be arranged, and the acoustic wave can be emitted from each probe after the delay process. In such a case, the acoustic wave can be transmitted to an arbitrary position in the subject without being influenced by the refraction of the acoustic wave by the plate.

**Reference Signs List**

| 11 | light source |
| 12 | light |
| 13 | optical component |
| 14 | light absorber |
| 15, 48, 62 | shape maintaining member |
| 16 | subject |
| 17 | acoustic wave |
| 18, 63 | probe |
| 19 | signal processor |
| 20 | image reconstruction processor |
| 70 | processing unit |
| 21 | display device |
| 22, 41, 43, 46 | virtual detection region |
| 25 | element |
| 26 | acoustic-wave receivable region |
| 31, 45, 49, 42 | probe |
| 44 | acoustic lens |
| 47 | filter |
| 50 | detected signal |
| 51 | opening |
| 61 | voxel of interest |

**Claims**

1.  A measuring apparatus comprising:

    a shape maintaining member (15, 48, 62) for maintaining a shape of at least a part of a subject (16);
    an element (18, 63) for receiving an acoustic wave (17) through the shape maintaining member (15, 48, 62) and for converting the acoustic wave (17) into a first electric signal; and
    a processing unit (19) adapted to generate image data using the first electric signal,
    wherein the processing unit (19) is adapted to determine, on the basis of the first electric signal, a first time from when the acoustic wave (17) is generated in the subject (16) to when the acoustic wave (17) reaches a reception surface of the element (18, 63), **characterized in that**
    the acoustic wave (17) is generated in the subject (16) when the subject (16) is irradiated with light; and
    the processing unit (19) is adapted to convert the first electric signal into a second electric signal using a difference between the first time and a second time in which the acoustic wave (17) travels from a virtual detection region (22, 41, 43, 46) near a subject-side surface of the shape maintaining member (15, 48, 62) to the reception surface of the element (18, 63), the second electric signal being assumed to have been obtained by receiving the acoustic wave (17) at the virtual detection region (22, 41, 43, 46).

2.  A measuring apparatus comprising:

a shape maintaining member (15, 48, 62) for maintaining a shape of at least a part of a subject (16);

an element (18, 63) for receiving a reflected acoustic wave (17) through the shape maintaining member (15, 48, 62) and for converting the reflected acoustic wave (17) into a first electric signal, the reflected acoustic wave (17) being reflected in the subject (16) when a transmission acoustic wave is transmitted toward the subject (16); and

a processing unit (19) adapted to generate image data using the first electric signal,

wherein the processing unit (19) is adapted to determine, on the basis of the first electric signal, a first time which is one-half of a time from when the element (18, 63) transmits the transmission acoustic wave to when the element receives the reflected acoustic wave (17), **characterized in that**

the processing unit (19) is adapted to convert the first electric signal into a second electric signal using a difference between the first time and a second time in which the reflected acoustic wave (17) travels from a virtual detection region (22, 41, 43, 46) near a subject-side surface of the shape maintaining member (15, 48, 62) to the reception surface of the element (18, 63), the second electric signal being assumed to have been obtained by receiving the reflected acoustic wave (17) at the virtual detection region (22, 41, 43, 46).

3. The measuring apparatus according to Claim 1 or 2,

wherein the element (18, 63) is provided with an acoustic lens (44) on the reception surface or is formed such that the reception surface is concave, and

wherein the virtual detection region is a region where a distance from the element (42) is acoustically constant.

4. The measuring apparatus according to Claim 1 or 2,

wherein the shape maintaining member (48) includes a filter (47) having an opening (51) that defines the virtual detection region near the subject-side surface of the shape maintaining member (48),

wherein the element (49) is divided into a plurality of the reception surfaces at which the acoustic wave is received and converted into a plurality of electric signals, one of the reception surfaces serving as a reference reception surface,

wherein the processing unit (19) is adapted to calculate the first electric signal by adding the plurality of electric signals after performing a delay process for the plurality of electric signals using the electric signal at the reference reception surface as a reference, and

wherein the processing unit (19) is adapted to set the second time to a time in which the acoustic wave travels from the virtual detection region to the reference reception surface.

5. The measuring apparatus according to one of Claims 1 to 4,

wherein the element (25) is provided in a plurality,

wherein the virtual detection region is set for each element (25), and

wherein the processing unit is adapted to generate the image data using a plurality of the second electric signals.

**Patentansprüche**

1. Messvorrichtung mit:

einem Formerhaltebauteil (15, 48, 62) zum Erhalten einer Form von zumindest einem Teil eines Subjekts (16);

einem Element (18, 63) zum Empfangen einer akustischen Welle (17) durch das Formerhaltebauteil (15, 48, 62) und zum Umwandeln der akustischen Welle (17) in ein erstes elektrisches Signal; und

einer Verarbeitungseinheit (19), die angepasst ist, Bilddaten unter Verwendung des ersten elektrischen Signals zu erzeugen,

wobei die Verarbeitungseinheit (19) angepasst ist, auf der Basis des ersten elektrischen Signals eine erste Zeit von dann, wenn die die akustische Welle (17) in dem Subjekt erzeugt wird, bis dahin, wenn die akustische Welle (17) eine Empfangsfläche des Elements (18, 63) erreicht, zu bestimmen, **dadurch gekennzeichnet, dass**

die akustische Welle (17) in dem Subjekt (16) erzeugt wird, wenn das Subjekt (16) mit Licht bestrahlt wird; und

die Verarbeitungseinheit (19) angepasst ist, das erste elektrische Signal in ein zweites elektrisches Signal unter Verwendung einer Differenz zwischen der ersten Zeit und einer zweiten Zeit, in der sich die a-kustische Welle (17) von einem virtuellen Erfassungsbereich (22, 41, 43, 46) nahe an einer subjektseitigen Oberfläche des Formerhaltebauteils (15, 48, 62) zu der Empfangsfläche des Elements (18, 63) bewegt, wobei angenommen wird, dass das zweite elektrische Signal durch Empfangen der akustischen Welle (17) an dem virtuellen Erfassungsbereich (22, 41, 43, 46) erhalten wurde.

2. Messvorrichtung mit:

einem Formerhaltebauteil (15, 48, 62) zum Erhalten einer Form von zumindest einem Teil eines Subjekts (16);
einem Element (18, 63) zum Empfangen einer reflektierten akustischen Welle (17) durch das Formerhaltebauteil (15, 48, 62) und zum Umwandeln der reflektierten akustischen Welle (17) in ein erstes elektrisches Signal, wobei die reflektierte Welle (17) in dem Subjekt reflektiert wird, wenn eine akustische Transmissionswelle zu dem Subjekt (16) übertragen wird; und
eine Verarbeitungseinheit (19), die angepasst ist, Bilddaten unter Verwendung des ersten elektrischen Signals zu erzeugen,
wobei die Verarbeitungseinheit (19) angepasst ist, auf der Basis des ersten elektrischen Signals eine erste Zeit, die die Hälfte einer Zeit von dann, wenn das Element (18, 63) die akustische Transmissionswelle transmittiert, bis dahin, wenn das Element die reflektierte akustische Welle empfängt (17), ist, zu bestimmen, **dadurch gekennzeichnet, dass**
die Verarbeitungseinheit (19) angepasst ist, das erste elektrische Signal in ein zweites elektrisches Signal unter Verwendung einer Differenz zwischen der ersten Zeit und einer zweiten Zeit umzuwandeln, in der die reflektierte akustische Welle (17) sich von einem virtuellen Erfassungsbereich (22, 41, 43, 46) nahe an einer subjektseitigen Oberfläche des Formerhaltebauteils (15, 48, 62) hin zu der Empfangsfläche des Elements (18, 63) bewegt, wobei angenommen wird, dass das zweite elektrische Signal durch Empfangen der reflektierten akustischen Welle (17) an dem virtuellen Erfassungsbereich (22, 41, 43, 46) erhalten wird.

3. Messvorrichtung nach Anspruch 1 oder 2,
wobei das Element (18, 63) mit einer akustischen Linse (44) auf der Empfangsfläche versehen ist, oder so geformt ist, dass die Empfangsfläche konkav ist, und
wobei der virtuelle Erfassungsbereich ein Bereich ist, an dem ein Abstand von dem Element (42) akustisch konstant ist.

4. Messvorrichtung nach Anspruch 1 oder 2,
wobei das Formerhaltebauteil (48) einen Filter (47) mit einer Öffnung (51) enthält, der den virtuellen Erfassungsbereich nahe der subjektseitigen Oberfläche des Formerhaltebauteils (48) definiert,
wobei das Element (49) in eine Vielzahl von Empfangsoberflächen eingeteilt ist, an denen die akustische Welle empfangen und in eine Vielzahl von elektrischen Signalen konvertiert wird, wobei eine der Empfangsoberflächen als eine Referenzempfangsoberfläche dient,
wobei die Verarbeitungseinheit (19) angepasst ist, das erste elektrische Signal durch Addieren der Vielzahl von elektrischen Signalen nach Durchführen eines Verzögerungsprozesses für die Vielzahl von elektrischen Signalen unter Verwendung des elektrischen Signals an der Referenzempfangsoberfläche als einer Referenz zu berechnen, und
wobei die Verarbeitungseinheit (19) angepasst ist, die zweite Zeit als eine Zeit einzustellen, in der sich die akustische Welle von dem virtuellen Erfassungsbereich zu der Referenzempfangsfläche bewegt.

5. Messvorrichtung nach einem der Ansprüche 1 bis 4,
wobei das Element (25) in einer Vielzahl bereitgestellt ist,
wobei der virtuelle Empfangsbereich für jedes Element (25) eingestellt ist, und
wobei die Verarbeitungseinheit angepasst ist, die Bilddaten unter Verwendung einer Vielzahl von zweiten elektrischen Signalen zu erzeugen.

**Revendications**

1. Appareil de mesure comprenant :

un élément de maintien de forme (15, 48, 62) pour maintenir une forme d'au moins une partie d'un sujet (16) ;
un élément (18, 63) pour recevoir une onde acoustique (17) à travers l'élément de maintien de forme (15, 48, 62) et pour convertir l'onde acoustique (17) en un premier signal électrique ; et
une unité de traitement (19) adaptée pour générer des données d'image en utilisant le premier signal électrique, dans lequel l'unité de traitement (19) est adaptée pour déterminer, sur la base du premier signal électrique, un premier temps entre le moment où l'onde acoustique (17) est générée chez le sujet (16) et celui où l'onde acoustique (17) atteint une surface de réception de l'élément (18, 63), **caractérisé en ce que**
l'onde acoustique (17) est générée chez le sujet (16) lorsque le sujet (16) est irradié avec de la lumière ; et
l'unité de traitement (19) est adaptée pour convertir le premier signal électrique en un deuxième signal électrique en utilisant une différence entre le premier temps et un deuxième temps où l'onde acoustique (17) se déplace à partir d'une région de détection virtuelle (22, 41, 43, 46) à proximité d'une surface côté sujet de l'élément de maintien de forme (15, 48, 62) jusqu'à la surface de réception de l'élément (18, 63), le deuxième signal électrique étant censé être obtenu par la réception de l'onde acoustique (17) au niveau de la région de détection virtuelle (22, 41, 43, 46).

2. Appareil de mesure comprenant :

un élément de maintien de forme (15, 48, 62) pour maintenir une forme d'au moins une partie d'un sujet (16) ;
un élément (18, 63) pour recevoir une onde acoustique réfléchie (17) à travers l'élément de maintien de forme (15, 48, 62) et pour convertir l'onde acoustique réfléchie (17) en un premier signal électrique, l'onde acoustique réfléchie (17) étant réfléchie chez le sujet (16) lorsqu'une onde acoustique de transmission est transmise vers le sujet (16) ; et
une unité de traitement (19) adaptée pour générer des données d'image en utilisant le premier signal électrique, dans lequel l'unité de traitement (19) est adaptée pour déterminer, sur la base du premier signal électrique, un premier temps qui est la moitié d'un temps entre le moment où l'élément (18, 63) transmet l'onde acoustique de transmission et celui où l'élément reçoit l'onde acoustique réfléchie (17), **caractérisé en ce que**
l'unité de traitement (19) est adaptée pour convertir le premier signal électrique en un deuxième signal électrique en utilisant une différence entre le premier temps et un deuxième temps où l'onde acoustique réfléchie (17) se déplace à partir d'une région de détection virtuelle (22, 41, 43, 46) à proximité d'une surface côté sujet de l'élément de maintien de forme (15, 48, 62) jusqu'à la surface de réception de l'élément (18, 63), le deuxième signal électrique étant censé être obtenu par la réception de l'onde acoustique réfléchie (17) au niveau de la région de détection virtuelle (22, 41, 43, 46).

3. Appareil de mesure selon la revendication 1 ou 2,
dans lequel l'élément (18, 63) est pourvu d'une lentille acoustique (44) sur la surface de réception ou est formé de sorte que la surface de réception soit concave, et
dans lequel la région de détection virtuelle est une région où une distance à partir de l'élément (42) est acoustiquement constante.

4. Appareil de mesure selon la revendication 1 ou 2,
dans lequel l'élément de maintien de forme (48) comporte un filtre (47) ayant une ouverture (51) qui définit la région de détection virtuelle à proximité de la surface côté sujet de l'élément de maintien de forme (48),
dans lequel l'élément (49) est divisé en une pluralité de surfaces de réception au niveau desquelles l'onde acoustique est reçue et convertie en une pluralité de signaux électriques, l'une des surfaces de réception servant de surface de réception de référence,
dans lequel l'unité de traitement (19) est adaptée pour calculer le premier signal électrique par l'addition de la pluralité de signaux électriques suite à la mise en oeuvre d'un processus de retard pour la pluralité de signaux électriques en utilisant le signal électrique au niveau de la surface de réception de référence en tant que référence, et
dans lequel l'unité de traitement (19) est adaptée pour régler le deuxième temps à un temps où l'onde acoustique se déplace à partir de la région de détection virtuelle jusqu'à la surface de réception de référence.

5. Appareil de mesure selon l'une des revendications 1 à 4,
dans lequel l'élément (25) est prévu en pluralité, dans lequel la région de détection virtuelle est définie pour chaque élément (25), et
dans lequel l'unité de traitement est adaptée pour générer les données d'image en utilisant une pluralité de deuxièmes signaux électriques.

[Fig. 1A]

[Fig. 1B]

[Fig. 2A]

[Fig. 2B]

[Fig. 3A]

41

d

42

[Fig. 3B]

43

d

44

45

[Fig. 3C]

43

44

45

[Fig. 4]

[Fig. 5]

[Fig. 6A]

[Fig. 6B]

[Fig. 6C]

[Fig. 6D]

[Fig. 7]

SOUND
VELOCITY: $c_2$

SOUND
VELOCITY: $c_1$

**EP 2 482 713 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6607489 B
- WO 2010074103 A1
- WO 2010074104 A1
- US 20100094561 A1